# EUROPEAN PATENT APPLICATION

(11) **EP 1 953 221 A2**
(43) Date of publication of application: **06.08.2008**
(21) Application number: 07022903.4
(22) Date of filing: 26.11.2007
(51) Int. Cl.: C12N 9/02, C12N 15/09, C12N 5/10

(54) **Bilirubin oxidase mutant having thermal stability**

(30) Priority: 07.12.2006 JP 2006330352; 19.06.2007 JP 2007160964
(71) Applicant: Sony Corporation, Minato-ku Tokyo 108-0075 (JP)
(72) Inventor: Kumita, Hideyuki, Tokyo 108-0075 (JP); Tokita, Yuichi, Tokyo 108-0075 (JP); Goto, Yoshio, Tokyo 108-0075 (JP)
(74) Representative: Müller - Hoffmann & Partner

(57) **Abstract**

A heat-resistant bilirubin oxidase mutant is disclosed. The bilirubin oxidase is obtained by deletion, replacement, addition or insertion of at least one amino acid residue of a wild type amino sequence of SEQ. ID. No. 1 of a bilirubin oxidase derived from an imperfect filamentous fungus, *Myrothecium verrucaria.*

## Description

### CROSS REFERENCES TO RELATED APPLICATIONS

The present invention contains subject matter related to Japanese Patent Applications JP 2006-330352 and JP 2007-160964 filed in the Japan Patent Office on December 7, 2006 and June 19, 2007, respectively, the entire contents of which being incorporated herein by reference.

### BACKGROUND OF THE INVENTION

### 1. Field of the Invention

The present invention relates to a bilirubin oxidase mutant having thermal stability. More specifically, the present invention relates to a bilirubin oxidase mutant having prescribed levels or more of heat resistance in addition to enzymatic activity.

### 2. Description of the Related Art

An "enzyme" is a biocatalyst for allowing many reactions relative to the maintenance of life to smoothly proceed under a mild condition *in vivo.* This enzyme turns over *in vivo*, is produced *in vivo* depending on the situation and exhibits its catalytic function.

At present, technologies for utilizing this enzyme *in vitro* have already been put into practical use or studied towards practical implementation. For example, a technology for utilizing an enzyme has been developed in various technical fields such as the production of a useful substance, the production, measurement or analysis of energy-related substance, the environmental preservation and the medical treatment. In relatively recent years, technologies regarding an enzyme cell which is one kind of a fuel cell (see, for example, JP-A-2004-71559 (Patent Document 1)), an enzyme electrode, an enzyme sensor (a sensor for measuring a chemical substance utilizing an enzymatic reaction) and the like have also been proposed.

Since a chemical main body of this enzyme is a protein, the enzyme has properties that it is denatured by the degree of heat or pH. For that reason, enzymes have low stability *in vitro* as compared with other chemical catalysts such as metal catalysts. Accordingly, when an enzyme is utilized *in vitro*, it is important to allow the enzyme to work more stably in response to an environmental change and to maintain an activity thereof.

When an enzyme is utilized *in vitro*, approaches such as a method for artificially modifying the nature or function of the enzyme itself and a method for devising the environment of a site where the enzyme works are employed. With respect to the former method, it is generally carried out that the base sequence of a gene encoding a protein is artificially modified, the thus modified gene is expressed in an organism such as *Escherichia coli* to produce an artificially mutated protein, and the protein mutant having functions and natures adapted to the use purpose is then subjected to separation (screening) (see, for example, JP-A-2004-298185 (Patent Document 2)).

The "bilirubin oxidase" as referred to herein is an enzyme which catalyzes a reaction for oxidizing bilirubin into biliverdin and is one kind of enzyme belonging to a multicopper oxidase (a general term of an enzymes having plural copper ions in the active center) . This enzyme has hitherto been widely used as an inspection reagent of liver function and the like (a measurement reagent of bilirubin in a blood serum) in the clinical laboratory examination. In recent years, this enzyme is also regarded as a catalyst for realizing an electrochemical four-electron reduction reaction of oxygen on a cathode side of the foregoing enzyme cell.

Under circumstances where expectations for utilizing this bilirubin oxidase *in vitro* are rising, a technology for investigating the same enzyme having more excellent thermal stability (see, for example, JP-A-2006-68003 (Patent Document 3)) and a technology for stably maintaining the enzymatic activity of the same enzyme over a longer period of time (see, for example, JP-A-2000-83661 (Patent Document 4)) have also been proposed.

### SUMMARY OF THE INVENTION

In consideration of the utilization of a bilirubin oxidase *in vitro,* it is necessary that the thermal stability is more enhanced. However, this bilirubin oxidase involves a problem that the enzymatic activity is reduced to not more than 20 % by heating at 60°C for one hour. For example, in the field of an enzyme cell, since the bilirubin oxidase has the lowest thermal stability among a group of enzymes to be utilized and is remarkably low in the thermal stability as compared with enzymes on an anode side (for example, glucose dehydrogenase and diaphorase), it is not suitable to put an enzyme cell into practical use. Also, though there is a choice to substitute this bilirubin oxidase with laccase which is a multicopper oxidase, this laccase involves not only a problem regarding the heat resistance but a problem that the enzymatic activity at room temperature in a neutral pH region is remarkably low as compared with the bilirubin oxidase.

Then, in consideration of the wide applicability of a bilirubin oxidase *in vitro,* it is desirable to provide a bilirubin oxidase mutant having prescribed levels or more of enzymatic activity and heat resistance of a bilirubin oxidase.

According to an embodiment of the present invention, there is provided a heat-resistant bilirubin oxidase mutant obtained by deletion, replacement, addition or insertion of at least one amino acid residue of the wild type amino sequence of SEQ. ID. No. 1 of a bilirubin oxidase derived from, an imperfect filamentous fungus, *Myrothecium verrucaria* (hereinafter referred to as *"M. verrucaria"*) so as to have enhanced heat resistance, and more favorably a heat-resistant bilirubin oxidase mutant having, for example, a denaturation temperature Tₘ value of 72°C or higher. Furthermore, there is provided a heat-resistant bilirubin oxidase mutant in which a residual activity after heating at 60°C for one hour is 20 % or more. For example, there is provided a heat-resistant bilirubin oxidase mutant having amino acid sequences of SEQ. ID. Nos. 2 to 45 and 57 to 67. As the foregoing imperfect filamentous fungus, for example, a strain of *M. verrucaria* NBRC (IFO) 6113 can be employed. Also, when the heat-resistant bilirubin oxidase mutant is expressed by using a yeast, *Pichia methanolica* as a host, it is possible to achieve abundant expression.

Here, in a heat-resistant bilirubin oxidase mutant represented by SEQ. ID. No. 2, glutamine at the 49th position from the N-terminus of the wild type amino acid sequence of SEQ. ID. No. 1 is replaced with lysine (hereafter abbreviated as "Q49K") . Similarly, in a heat-resistant bilirubin oxidase mutant represented by SEQ. ID. No. 3, glutamine at the 72nd position is replaced with glutamic acid (hereafter abbreviated as "Q72E"); in a heat-resistant bilirubin oxidase mutant represented by SEQ. ID. No. 4, valine at the 81st position is replaced with leucine (hereafter abbreviated as "V81L"); in a heat-resistant bilirubin oxidase mutant represented by SEQ. ID. No. 5, tyrosine at the 121st position is replaced with serine (hereafter abbreviated as "Y121S"); in a heat-resistant bilirubin oxidase mutant represented by SEQ. ID. No. 6, arginine at the 147th position is replaced with proline (hereafter abbreviated as "R147P"); in a heat-resistant bilirubin oxidase mutant represented by SEQ. ID. No. 7, alanine at the 185th position is replaced with serine (hereafter abbreviated as "A185S"); in a heat-resistant bilirubin oxidase mutant represented by SEQ. ID. No. 8, proline at the 210th position is replaced with leucine (hereafter abbreviated as "P210L"); in a heat-resistant bilirubin oxidase mutant represented by SEQ. ID. No. 9, phenylalanine at the 225th position is replaced with valine (hereafter abbreviated as "F225V"); in a heat-resistant bilirubin oxidase mutant represented by SEQ. ID. No. 10, glycine at the 258th position is replaced with valine (hereafter abbreviated as "G258V"); in a heat-resistant bilirubin oxidase mutant represented by SEQ. ID. No. 11, alanine at the 264th position is replaced with valine (hereafter abbreviated as "A264V"); in a heat-resistant bilirubin oxidase mutant represented by SEQ. ID. No. 12, aspartic acid at the 322nd position is replaced with asparagine (hereafter abbreviated as "D322N"); in a heat-resistant bilirubin oxidase mutant represented by SEQ. ID. No. 13, asparagine at the 335th position is replaced with serine (hereafter abbreviated as "N335S"); in a heat-resistant bilirubin oxidase mutant represented by SEQ. ID. No. 14, arginine at the 356th position is replaced with leucine (hereafter abbreviated as "R356L"); in a heat-resistant bilirubin oxidase mutant represented by SEQ. ID. No. 15, proline at the 359th position is replaced with serine (hereafter abbreviated as "P359S"); in a heat-resistant bilirubin oxidase mutant represented by SEQ. ID. No. 16, aspartic acid at the 370th position is replaced with tyrosine (hereafter abbreviated as "D370Y"); in a heat-resistant bilirubin oxidase mutant represented by SEQ. ID. No. 17, valine at the 371st position is replaced with alanine (hereafter abbreviated as "V371A"); in a heat-resistant bilirubin oxidase mutant represented by SEQ. ID. No. 18, proline at the 423rd position is replaced with leucine (hereafter abbreviated as "P423L"); in a heat-resistant bilirubin oxidase mutant represented by SEQ. ID. No. 19, methionine at the 468th position is replaced with valine (hereafter abbreviated as "M468V"); in a heat-resistant bilirubin oxidase mutant represented by SEQ. ID. No. 20, leucine at the 476th position is replaced with proline (hereafter abbreviated as "L476P"); and in a heat-resistant bilirubin oxidase mutant represented by SEQ. ID. No. 21, valine at the 513rd position is replaced with leucine (hereafter abbreviated as "V513L"). Also, in a heat-resistant bilirubin oxidase mutant represented by SEQ. ID. No. 57, alanine at the 103rd position is replaced with proline (hereafter abbreviated as "A103P"); in a heat-resistant bilirubin oxidase mutant represented by SEQ. ID. No. 58, tyrosine at the 270th position is replaced with aspartic acid (hereafter abbreviated as "Y270D"); in a heat-resistant bilirubin oxidase mutant represented by SEQ. ID. No. 59, serine at the 299th position is replaced with asparagine (hereafter abbreviated as "S299N"); in a heat-resistant bilirubin oxidase mutant represented by SEQ. ID. No. 60, valine at the 381st position is replaced with leucine (hereafter abbreviated as "V381L"); in a heat-resistant bilirubin oxidase mutant represented by SEQ. ID. No. 61, alanine at the 418th position is replaced with threonine (hereafter abbreviated as "A418T"); and in a heat-resistant bilirubin oxidase mutant represented by SEQ. ID. No. 62, arginine at the 437th position is replaced with histidine (hereafter abbreviated as "R437H").

Also, in a heat-resistant bilirubin oxidase mutant represented by SEQ. ID. No. 22, glutamine at the 49th position from the N-terminus of the wild type amino acid sequence of SEQ. ID. No. 1 is replaced with lysine, and valine at the 371st position is replaced with alanine (hereafter abbreviated as "Q49K/V371A"). Similarly, in a heat-resistant bilirubin oxidase mutant represented by SEQ. ID. No. 23, glutamine at the 72nd position is replaced with glutamic acid, and proline at the 210th position is replaced with leucine (hereafter abbreviated as "Q72E/P210L"); in a heat-resistant bilirubin oxidase mutant represented by SEQ. ID. No. 24, glutamine at the 72nd position is replaced with glutamic acid, and alanine at the 264th position is replaced with valine (hereafter abbreviated as "Q72E/A264V"); in a heat-resistant bilirubin oxidase mutant represented by SEQ. ID. No. 25, valine at the 81st position is replaced with leucine, and arginine at the 147th position is replaced with proline (hereafter abbreviated as "V81L/R147P"); in a heat-resistant bilirubin oxidase mutant represented by SEQ. ID. No. 26, valine at the 81st position is replaced with leucine, and proline at the 423rd position is replaced with leucine (hereafter abbreviated as "V81L/P423L"); in a heat-resistant bilirubin oxidase mutant represented by SEQ. ID. No. 27, tyrosine at the 121st position is replaced with serine, and leucine at the 476th position is replaced with proline (hereafter abbreviated as "Y121S/L476P"); in a heat-resistant bilirubin oxidase mutant represented by SEQ. ID. No. 28, alanine at the 185th position is replaced with serine, and glycine at the 258th position is replaced with valine (hereafter abbreviated as "A185S/G258V"); in a heat-resistant bilirubin oxidase mutant represented by SEQ. ID. No. 29, proline at the 210th position is replaced with leucine, and alanine at the 264th position is replaced with valine (hereafter abbreviated as "P210L/A264V"); in a heat-resistant bilirubin oxidase mutant represented by SEQ. ID. No. 30, phenylalanine at the 225th position is replaced with valine, and aspartic acid at the 322nd position is replaced with asparagine (hereafter abbreviated as "F225V/D322N"); in a heat-resistant bilirubin oxidase mutant represented by SEQ. ID. No. 31, phenylalanine at 225th position is replaced by valine, and leucine at the 476th position is replaced with proline (hereafter abbreviated as "F225V/L476P"); in a heat-resistant bilirubin oxidase mutant represented by SEQ. ID. No. 32, alanine at the 264th position is replaced with valine, and arginine at the 356th position is replaced with leucine (hereafter abbreviated as "A264V/R356L"); in a heat-resistant bilirubin oxidase mutant represented by SEQ. ID. No. 33, alanine at the 264th position is replaced with valine, and leucine at the 476th position is replaced with proline (hereafter abbreviated as "A264V/L476P"); in a heat-resistant bilirubin oxidase mutant represented by SEQ. ID. No. 34, aspartic acid at the 322nd position is replaced with asparagine, and methionine at the 468th position is replaced with valine (hereafter abbreviated as "D322N/M468V"); in a heat-resistant bilirubin oxidase mutant represented by SEQ. ID. No. 35, asparagine at the 335th position is replaced with serine, and proline at the 423rd position is replaced with leucine (hereafter abbreviated as "N335S/P423L"); in a heat-resistant bilirubin oxidase mutant represented by SEQ. ID. No. 36, arginine at the 356th position is replaced with leucine, and leucine at the 476th position is replaced with proline (hereafter abbreviated as "R356L/L476P"); and in a heat-resistant bilirubin oxidase mutant represented by SEQ. ID. No. 37, valine at the 371st position is replaced with alanine, and valine at the 513rd position is replaced with leucine (hereafter abbreviated as "V371A/V513L").

Furthermore, in a heat-resistant bilirubin oxidase mutant represented by SEQ. ID. No. 38, glutamine at the 49th position from the N-terminus of the wild type amino acid sequence of SEQ. ID. No. 1 is replaced with lysine, valine at the 371st position is replaced with alanine, and valine at the 513rd position is replaced with leucine (hereafter abbreviated as "Q49K/V371A/V513L"). Similarly, in a heat-resistant bilirubin oxidase mutant represented by SEQ. ID. No. 39, glutamine at the 72nd position is replaced with glutamic acid, proline at the 210th position is replaced with leucine, and alanine at the 264th position is replaced with valine (hereafter abbreviated as "Q72E/P210L/A264V"); in a heat-resistant bilirubin oxidase mutant represented by SEQ. ID. No. 40, valine at the 81st position is replaced with leucine, asparagine at the 335th position is replaced with serine, and proline at the 423rd position is replaced with leucine (hereafter abbreviated as "V81L/N335S/P423L"); in a heat-resistant bilirubin oxidase mutant represented by SEQ. ID. No. 41, tyrosine at the 121st position is replaced with serine, aspartic acid at the 370th position is replaced with tyrosine, and leucine at the 476th position is replaced with proline (hereafter abbreviated as "Y121S/D370Y/L476P"); in a heat-resistant bilirubin oxidase mutant represented by SEQ. ID. No. 42, alanine at the 185th position is replaced with serine, alanine at the 264th position is replaced with valine, and leucine at the 476th position is replaced with proline (hereafter abbreviated as "A185S/A264V/L476P"); in a heat-resistant bilirubin oxidase mutant represented by SEQ. ID. No. 43, phenylalanine at the 225th position is replaced with valine, aspartic acid at the 322nd position is replaced with asparagine, and methionine at the 468th position is replaced with valine (hereafter abbreviated as "F225V/D322N/M468V"); in a heat-resistant bilirubin oxidase mutant represented by SEQ. ID. No. 44, phenylalanine at the 225th position is replaced with valine, aspartic acid at the 370th position is replaced with tyrosine, and leucine at the 476th position is replaced with proline (hereafter abbreviated as "F225V/D370Y/L476P"); and in a heat-resistant bilirubin oxidase mutant represented by SEQ. ID. No. 45, alanine at the 264th position is replaced with valine, arginine at the 356th position is replaced with leucine, and leucine at the 476th position is replaced with proline (hereafter abbreviated as "A264V/R356L/L476P"). Also, in a heat-resistant bilirubin oxidase mutant represented by SEQ. ID. No. 63, alanine at the 264th position is replaced with valine, serine at the 299th position is replaced with asparagine, and leucine at the 476th position is replaced with proline (hereinafter abbreviated as "A264V/S299N/L476P"); in a heat-resistant bilirubin oxidase mutant represented by SEQ. ID. No. 64, alanine at the 264th position is replaced with valine, valine at the 381st position is replaced with leucine, and leucine at the 476th position is replaced with proline (hereinafter abbreviated as "A264V/V381L/L476P"); in a heat-resistant bilirubin oxidase mutant represented by SEQ. ID. No. 65, alanine at the 264th position is replaced with valine, alanine at the 418th position is replaced with threonine, and leucine at the 476th position is replaced with proline (hereinafter abbreviated as "A264V/A418T/L476P"); and in a heat-resistant bilirubin oxidase mutant represented by SEQ. ID. No. 66, alanine at the 264th position is replaced with valine, arginine at the 437th position is replaced with histidine, and leucine at the 476th position is replaced with proline (hereinafter abbreviated as "A264V/R437H/L476P"). Furthermore, in a heat-resistant bilirubin oxidase mutant represented by SEQ. ID. No. 67, alanine at the 103rd position is replaced with proline, alanine at the 264th position is replaced with valine, tyrosine at the 270th position is replaced with aspartic acid, and leucine at the 476th position is replaced with proline (hereinafter abbreviated as "A103P/A264V/Y270D/L476P").

The term "residual enzyme activity after heating" as referred to herein may be referred to as "residual enzymatic activity" or "retention of enzymatic activity" and is a value representing a change in activity before and after an enzyme is subjected to prescribed heating. That is, the residual activity is a value of percentage representing how the activity value after heating has changed as compared with that before heating upon the measurement of enzymatic activity under the same condition. The condition of the term "heating" as referred to herein is a stationary treatment in a buffer solution at 60°C for one hour, and a ratio of the foregoing enzymatic activity value before and after this heating is represented by percentage.

Also, the term "denaturation temperature Tₘ" as referred to herein is a value determined by the measurement by differential scanning microcalorimetry. A temperature rise rate of an enzyme solution as a preparation in this measure was set up at 60°C per hour.

The heat-resistant bilirubin oxidase mutant according to an embodiment of the present invention is able to maintain the enzymatic activity in a prescribed level or more even after heating.

### BRIEF DESCRIPTION OF THE DRAWINGS

Fig. 1 is a diagram showing one example of thermal stabilization screening and showing the behavior of color generation of ABTS (one hour after the start of the reaction) .

Fig. 2 is a diagram showing a UV-vis spectrum of a recombinant BO mutant.

### DESCRIPTION OF THE PREFERRED EMBODIMENTS

Next, specific examples according to an embodiment of the present invention are described on the basis of the experimental results.

### (Example 1) cDNA cloning of BO derived from M. verrucaria 1-1. Culture of M. verrucaria and isolation of messenger RNA:

A strain of *M. verrucaria* NBRC (IFO) 6113 used in the present Example was purchased from National Institute of Technology and Evaluation, Department of Biotechnology. The obtained lyophilizate was suspended in a condensate (polypeptone: 0.5 %, yeast extract: 0.3 %, MgSO₄·7H₂O: 0.1 %), and this suspension was inoculated on a potato dextrose agar (PDA) plate (potato dextrose: 2.4 %, agarose: 1.5 %). As a result of culture at room temperature for 5 to 7 days, the surface of the PDA plate was covered by a white hypha. This was scraped by a spatula and preserved at -80°C. The yield of the bacterial cell was from 50 to 60 mg (wet weight) per PDA plate (diameter: 9 cm).

A messenger RNA (hereinafter referred to a "mRNA") was extracted as a total RNA (a mixture of mRNA, ribosomal RNA and transfer RNA) . The total RNA was obtained in an amount of 100 µg (quantitatively determined by UV absorption) from about 100 mg of the lyophilizate powder of *M. verrucaria,* and a 1/4 portion thereof was used as a template RNA of one reaction of the next reverse transcription PCR.

### 1-2. Preparation of BO gene fragment by reverse transcription PCR:

The reverse transcription PCR was carried out by using a OneStep RT-PCR kit (manufactured by Qiagen Corporation) and using the foregoing total RNA as a template. A PCR primer to be used for the reverse transcription PCR was designed as shown in the following Table 1 on the basis of a previously reported base sequence of cDNA of BO.

**Table 1**

| | |
|---|---|
| N-Terminus side, *Hind*III (AAGCTT) site inserted | |
| | 5'-GGGAAGCTTATGTTCAAACACACACTTGGAGCTG-3' (SEQ. ID. No. 46) |
| C-Terminus side, *Xba*I (TCTAGA) site inserted | |
| | 5'-GGGTCTAGACTCGTCAGCTGCGGCGTAAGGTCTG-3' (SEQ. ID. No. 47) |

As a result of agarose gel electrophoresis of the resulting PCR product, a strong band could be verified in the vicinity of 1,700 bp. In view of the size of 1,700 bp, this fragment was estimated to be an amplified fragment containing the desired BO gene, and therefore, this fragment was cut out from the agarose gel slab and used in a next step.

### 1-3. Integration of BO gene fragment into pYES2/CT vector:

The obtained amplified fragment of 1, 700 bp was digested by restriction enzymes *Hind*III and *Xba*I and then coupled with a pYES2/CT plasmid vector (manufactured by Invitrogen Corporation) as digested by the same enzymes. On that occasion, an alkaline phosphatase derived from *Calf intestine* (manufactured by Takara Bio Inc.) was used for the dephosphorylation of a 5'-protruding end of the pYES2/CT vector by the restriction enzyme treatment, and T4 DNA ligase (manufactured by Takara Bio Inc.) was used for a coupling reaction between the inserted fragment and the pYES2/CT vector, respectively.

A strain of *E. coli* TOP10 (manufactured by Invitrogen Corporation) was transformed by the thus obtained reaction product and inoculated on an LB/Amp agar plate medium (having a composition as shown in Table 2). After culturing overnight, a colony of a transformant having drug resistance to ampicillin was obtained. This was cultured overnight on 3 mL of an LB/Amp medium, and the plasmid vector was isolated from the resulting bacterial cell.

**Table 2**

| | |
|---|---|
| Tryptophan | 1 % |
| Yeast extract | 0.5 % |
| Sodium chloride | 1 % |
| Ampicillin | 0.005 % |

As a result of examining the base sequence of the inserted portion containing a BO gene of the resulting plasmid vector, it was found to be SEQ. ID. No. 48.

The base sequence represented in SEQ ID. No. 48 is 1, 719 bp and is corresponding to 572 amino acid residues. On the other hand, a BO derived from *M. verrucaria* of a maturation type is constituted of 534 amino acid residues (SEQ. ID. No. 1). The 38 amino acid residues corresponding to a difference therebetween exists on the N-terminus side and are a signal peptide for governing the secretion of a protein existing on the C-terminus side. After translation, the portion is cleaved at the time of secretion.

### 1-4. Insertion of AAA sequence:

Next, with respect to the plasmid vector as prepared in 1-3, a part of the base sequence thereof was modified so as to increase the expression amount of the recombinant protein. Concretely, three bases on the upstream side (5'-side) relative to a start codon (ATG) were changed as follows.

**Table 3**

| | | |
|---|---|---|
| Before modification: | 5'-···ATTAAGAAATGTTCAAAC···-3' | (SED. ID. No. 49) |
| After modification: | 5'-···ATTAAGAAAATGTTCAAAC···-3 | (SED. ID. No. 50) |

The change of these three bases was carried out by a Quick-Change mutagenesis kit (manufactured by Stratagene Corporation) by using a PCR primer as shown in the following Table 4. The detailed experimental procedures followed those in a manual attached to the product.

**Table 4**

| |
|---|
| N-Terminus side: |
| 5'-CTATAGGGAATATTAAGAAAATGTTCAAACACACACTTG-3' (SED. ID. No. 51) |
| C-Terminus side: |
| 5'-CAAGTGTGTGTTTGAACATTTTCTTAATATTCCCTATAGTG-3' (SED. ID. No. 52) |

The verification of the base sequence was carried out in the entire region of the BO gene including the changed sites. As a result, it was verified that the base sequence was changed as designed. The plasmid vector after changing the sequence is hereinafter referred to as "pYES2/CT-BO vector".

### (Example 2) Construction of secretion expression system of recombinant BO by S. cerevisiae

### 2-1. Transformation of S. cerevisiae by pYES2/CT-BO vector:

Next, the transformation of S. *cerevisiae* was carried out by using the foregoing pYES2/CT-BO vector. As S. *cerevisiae,* a strain of INVSc1 (manufactured by Invitrogen Corporation) which is marketed along with the pYES2/CT vector was used. Here, the transformation of *S. cerevisiae* was carried out by a lithium acetate method. With respect to the detailed experimental procedures, a manual attached to the pYES2/CT vector was made by reference. For selecting the transformed yeast, an SCGlu agar plate medium (having a composition as shown in Table 2) was used.

**Table 5**

| | |
|---|---|
| Yeast nitrogen base (YNB) | 0.17 % |
| (NH₄)₂SO₄ | 0.5 % |
| L-Arginine | 0.01 % |
| L-Cysteine | 0.01 % |
| L-Leucine | 0.01 % |
| L-Lysine | 0.01 % |
| L-Threonine | 0.01 % |
| L-Tryptophan | 0.01 % |
| L-Aspartic acid | 0.005 % |
| L-Histidine | 0.005 % |
| L-Isoleucine | 0.005 % |
| L-Methionine | 0.005 % |
| L-Phenylalanine | 0.005 % |
| L-Proline | 0.005 % |
| L-Serine | 0.005 % |
| L-Tyrosine | 0.005 % |
| L-Valine | 0.005 % |
| Adenine | 0.01% |
| D-Glucose | 2 % |
| Agarose | 2 % |

### 2-2. Secretion expression of recombinant BO:

The colony of the transformant of *S*. *cerevisiae* by the pYES2/CT-BO vector was inoculated on 15 mL of an SCGlu liquid medium and cultured with shaking at 30°C for from 14 to 20 hours. The resulting bacterial cell was once precipitated by centrifugation (1,500 × g at room temperature for 10 minutes).

Here, after discarding the SCGlu liquid medium, the resulting bacterial cell was added in 50 mL of an SCGal medium (having a composition as shown in Table 6) such that a turbidity (OD₆₀₀) was about 0.5. This was cultured with shaking at 25°C for from 10 to 14 hours. After the culture, the bacterial cell was removed by centrifugation, the residual culture solution was concentrated to a degree of about 5 mL and dialyzed against a 20 mM sodium phosphate buffer solution (pH: 7.4).

**Table 6**

| | |
|---|---|
| Yeast nitrogen base (YNB) | 0.17 % |
| (NH₄)₂SO₄ | 0.5 % |
| L-Arginine | 0.01 % |
| L-Cysteine | 0.01 % |
| L-Leucine | 0.01 % |
| L-Lysine | 0.01 % |
| L-Threonine | 0.01 % |
| L-Tryptophan | 0.01 % |
| L-Aspartic acid | 0.005 % |
| L-Histidine | 0.005 % |
| L-Isoleucine | 0.005 % |
| L-Methionine | 0.005 % |
| L-Phenylalanine | 0.005 % |
| L-Proline | 0.005 % |
| L-Serine | 0.005 % |
| L-Tyrosine | 0.005 % |
| L-Valine | 0.005 % |
| Adenine | 0.01 % |
| D-Galactose | 2 % |
| Raffinose | 1% |
| Glycine | 1% |
| CuSO₄·5H₂O | 0.003 % |

The purification of the recombinant BO was carried out by Ni-NTA affinity chromatography (His-trap HP (1 mL), manufactured by Amersham Biosciences K.K.). The purification method followed that in a manual attached to the product. The recombinant BO obtained after the purification was verified to have a purity of 100 by SDS-PAGE or the like. The yield of the resulting recombinant BO was calculated into 1L-culture and found to be 0.36 mg.

### (Example 3) Thermal stabilization screening of recombinant BO by evolutionary molecular engineering method

Next, the recombinant BO was subjected to thermal stabilization screening by an evolutionary molecular engineering method. Concretely, the insertion of random mutation using Error-prone PCR, the preparation of a BO gene library as a transformant, the transformation of S. *cerevisiae* by the BO mutant gene library and the thermal stabilization screening by a 96-well plate were carried out.

### 3-1. Insertion of random mutation using Error-prone PCR:

The insertion of random mutation by Error-prone PCR was carried out by using the pYES2/CT-BO vector as a template. The PCR primer on the N-terminus side as used herein was designed so as to contain only one BglII side (AGATCT) existing in the downstream of the 218 base pairs relative to the start codon. Also, the C-terminus side was designed in the following manner so as to contain the *Xba*I site (TCTAGA) (see Table 7).

**Table 7**

| | |
|---|---|
| N-Terminus side, *Bgl*II (AGATCT) site inserted | |
| | 5'-GTAACCAATCCTGTGAATGGACAAGAGATCTGG-3' (SEQ. ID. No. 53) |
| C-Terminus side, *Xba*l (TCTAGA) site inserted | |
| | 5'-GGGATAGGCTTACCTTCGAAGGGCCCTCTAGACTC-3' (SEQ. ID. No. 54) |

The Error-prone PCR was carried out by a GeneMorph PCR mutagenesis kit (manufactured by Stratagene Corporation) by using this primer. With respect to the reaction condition, a manual attached to the same kit was made by reference.

As a result of agarose gel electrophoresis of the resulting PCR product, a PCR fragment of about 1, 500 bp could be obtained. The frequency of mutation as calculated from the yield of the resulting PCR product was 1.5 sites per 1,000 bp. With respect to the calculation method, a manual attached to the same kit was made by reference.

### 3-2. Preparation of BO gene library of mutant:

With respect to the BO gene fragment having mutation randomly inserted thereinto as prepared above in 3-1, integration of the pYES2/CT-BO vector into the *Bgl*II*-Xba*I sites and transformation of a strain of *E*. *coli* TOP10 were carried out in the same manner as described above in 1-3. Here, a plasmid library including about 6,600 transformant colonies, namely about 6,600 kinds of transformant genes.

### 3-3. Transformation of S. cerevisiae by transformant BO gene library:

The transformation of a strain of *S*. *cerevisiae* INVSc1 (manufactured by Invitrogen Corporation) by the transformant BO gene library was carried out in the same manner as described above in 3-2. A competent cell of *S*. *cerevisiae* INVSc1 was prepared by a lithium acetate method. The resulting transformant library was subjected to thermal stabilization screening by using a 96-well plate.

### 3-4. Thermal stabilization screening experiment using 96-well plate:

A 150-mL portion of an SCGlu medium was poured out into a 96-well plate. One colony of the thus prepared transformant yeast library was inoculated in each well. This was cultured with shaking at 27°C for from 20 to 23 hours. After this culture, the visual observation revealed that the turbidity of the respective wells became substantially constant.

At this stage, every 96-well plate was once subjected to centrifugation (1, 500 × g at 20°C for 10 minutes), thereby once precipitating the bacterial cell. The SCGlu medium was completely removed in such a manner that the bacterial cell precipitated on the bottom of each well was not disturbed. A 180-mL portion of an SCGal medium was poured out thereinto, and the bacterial cell was further cultured with shaking at 27°C for 8 hours. After this culture, the centrifugation (1,500 × g at 20°C for 10 minutes) was again carried out to precipitate the bacterial cell. 100 mL of this supernatant was transferred into a separate, new 96-well plate. Here, when carrying out heating, a sample solution on this 96-well plate was sealed by a cellophane tape and then allowed to stand in a dry oven at 80°C for 15 minutes. After heating, the sample solution was rapidly cooled on an ice bath for 5 minutes and then allowed to stand at room temperature for 15 minutes. An equal amount of a 20 mM ABTS solution (100 mM Tris-HCl, pH: 8.0) was mixed therewith. The situation that the solution in the well was colored green with the progress of reaction of ABTS was observed until one hour elapsed after the start of the reaction. Ones exhibiting strong coloration as compared with the wild type as a comparison were picked up, and bacterial cells corresponding thereto were preserved as 20 glycerol stocks at -80°C.

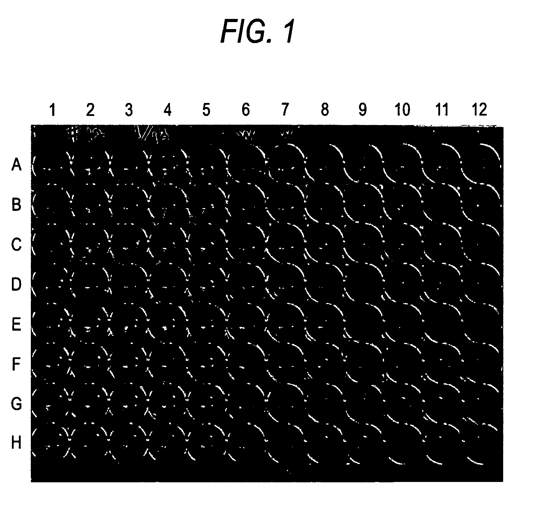

Fig. 1 shows one example of thermal stabilization screening. Fig. 1 shows the behavior of color generation of ABTS one hour after the start of the reaction. All of central two columns (6th and 7th columns from the left side) are concerned with the wild type recombinant BO as a comparison, in which the 6th column is concerned with one having been subjected to heating similar to other wells. The 7th column is concerned with the comparison in the case of the wild type recombinant BO not having been subjected to heating.

It is noted from Fig. 1 that the wells surrounded by a square cause strong color generation as compared with any of the wild types in the 6th column. It is thought that in these wells, a BO mutant having enhanced thermal stability is expressed as compared with the wild type recombinant BO.

In this Example 3, the thermal stabilization screening as described in 3-4 was performed with respect to 4, 000 samples in total in 50 sheets of a 96-well plate, and 26 transformant yeasts which are thought to have expressed the heat-resistant BO mutant were chosen.

Plasmid vectors were extracted with the obtained 26 transformant yeasts and subjected to an analysis of base sequence of the BO gene region. As a result, it became clear that the following 26 kinds of mutations were inserted into the BO gene. That is, mutations of the foregoing abbreviations Q49K, Q72E, V81L, Y121S, R147P, A185S, P210L, F225V, G258V, A264V, D322N, N335S, R356L, P359S, D370Y, V371A, P423L, M468V, L476P, V513L, A103P, Y270D, S299N, V381L, A418T and R437H were verified.

### (Example 4) Abundant expression by heat-resistant mutant Pichia methanolica

In the following, in order to achieve abundant expression of the 26 kinds of heat-resistant mutant candidacies discovered by the thermal stabilization screening, the construction of secretion expression system of recombinant BO using a yeast *Pichia methanolica* (hereinafter referred to as *"P. methanolica")* was newly performed, thereby attempting to achieve abundant expression of the wild type and heat-resistant mutant candidacies.

### 4-1. Preparation of pMETaB-BO vector and transformation of P. methanolica by this vector:

First of all, an expression vector to be used in an expression system of *P*. *methanolica* was prepared. Since a secretion signal: α-factor derived from *S*. *cerevisiae* is contained in a pMETaB vector (manufactured by Invitrogen Corporation), a gene corresponding to a maturation BO was inserted into its downstream. The amplification of the maturation BO gene region by PCR was carried out by using the pYES2/CT-BO vector as a template and using primers as shown in the following Table 8.

**Table 8**

| | |
|---|---|
| N-Terminus side, *Eco*RI (GAATTC) site inserted | |
| | 5'-GGGAATTCTTGCCCAGATCAGCCCACAGTATC-3' (SEQ. ID. No. 55) |
| C-Terminus side, Termination codon, *Sp*eI (ACTAGT) site inserted | |
| | 5'-GGGACTAGTCACTCGTCAGCTGCGGCGTAAGG-3' (SEQ. ID. No. 56) |

The obtained amplified fragment of 1, 500 bp was digested by restriction enzymes *Eco*RI and *Spe*I and then coupled with a pMETaB vector as digested by the same enzymes. On the occasion of this coupling reaction, the reaction product was subjected to the same treatment as that described above in 1-3. With respect to the thus prepared BO gene region-containing pMETaB vector (hereinafter referred to as "pMETaB-BO vector"), the verification of the base sequence of the inserted BO gene portion was carried out. In the case of the BO mutant, mutations were inserted into the thus prepared pMETaB-BO vector by QuickChange Mutagenesis Kits (manufactured by Invitrogen Corporation). The subsequent operations were similarly carried out irrespective of the wild type and the mutant.

In addition to the foregoing pMETaB-BO vectors of the wild type and 26 kinds of heat-resistant mutant candidacies, a pMETaB-BO vector of a multiple mutant obtained by combining two, three or four of the 26 kinds of heat-resistant mutant candidacies was similarly prepared and verified with respect to the base sequence.

The transformation of *P. methanolica* by all of the thus prepared pMETaB-BO vectors was carried out. A strain of PMAD11 (manufactured by Invitrogen Corporation) was used as *P. methanolica.* The transformation followed a method described in a manual attached to the pMETaB vector. The selection of the transformed yeast was carried out on an MD agar plate medium (having a composition as shown in Table 9). Competencies of this reaction were all up to 10/1 µg DNA and were substantially coincident with the values described in the manual.

**Table 9**

| | |
|---|---|
| Yeast nitrogen base (YNB) | 1.34 % |
| Biotin | 0.00004 % |
| D-Glucose | 2 % |
| Agarose | 1.5 % |

### 4-2. Abundant expression of recombinant BO by P. methanolica:

The colony of the transformant yeast on an MD medium as obtained 5 to 7 days after the transformation was cultured overnight on 3 mL of a BMDY medium (having a composition as shown in Table 10). A part of the resulting culture solution was again developed on an MD agar plate medium. A white purified colony obtained 2 to 3 days after this was used for the abundant expression in the next item.

**Table 10**

| | |
|---|---|
| Yeast extract | 1 % |
| Peptone | 2 % |
| Potassium phosphate buffer solution (pH: 6.0) | 100 mM |
| Yeast nitrogen base (YNB) | 1.34 % |
| Biotin | 0.00004 % |
| D-Glucose | 2 % |

Next, an operation of the abundant expression of recombinant BO by *P*. *methanolica* was carried out. The purified colony of the transformant yeast was inoculated on 50 mL of a BMDY liquid medium and cultured with shaking at 30°C overnight. At that time, the OD₆₀₀ was found to be from 2 to 5. The thus obtained bacterial cell was once precipitated by centrifugation (1, 500 × g at room temperature for 10 minutes), the BMDY liquid medium was removed, and only the bacterial cell was then suspended in 50 to 100 mL of a BMMY liquid medium (having a composition as shown in Table 11). The suspension was cultured with shaking at 27°C for 24 hours. Thereafter, methanol was added such that a final concentration was 0.5 %, and the mixture was further cultured under the same condition for 24 hours. After performing this until elapsing 96 hours, the bacterial cell was removed by centrifugation, and the residual culture solution was concentrated to a degree of about 5 to 10 mL and dialyzed against a 50 mM Tris-HCl buffer (pH: 7.6).

**Table 11**

| | |
|---|---|
| Yeast extract | 1 % |
| Peptone | 2 % |
| Potassium phosphate buffer solution (pH: 6.0) | 100 mM |
| Yeast nitrogen base (YNB) | 1.34 % |
| Biotin | 0.00004 % |
| Methanol | 0.5 % |
| CuSO₄·5H₂O | 0.003 % |

### 4-3. Purification of recombinant BO:

Subsequently, the purification of the recombinant BO by anion-exchange chromatography was carried out. A crude solution containing the recombinant BO as prepared in the preceding step was purified by using an anion-exchange column (HiTrap Q HP, bed volume: 5 mL, manufactured by GE Healthcare Bioscience Corp.). With respect to the purification condition, a previous report (Biochemistry, 38, 3034-3042 (1999)) was made by reference.

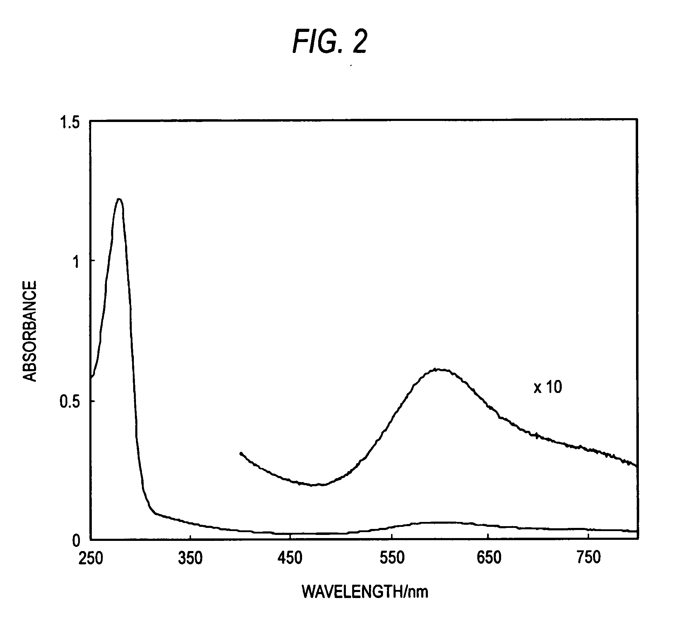

Next, the purification of the recombinant BO by hydrophobic chromatography was carried out. A column used for the hydrophobic chromatography is a Toyopearl Butyl-650 M column (100 mL, 20 mm × 20 cm, manufactured by Tosoh Corporation). With respect to the purification condition, a previous report (Biochemistry, 44, 7004-7012 (2005)) was made by reference. A UV-vis spectrum of the recombinant BO (A246V) obtained after the purification is shown in Fig. 2.

The spectral pattern of A264V as shown in Fig. 2 was completely coincident with that of a recombinant BO by *P*. *pastris* in a previous report (Protein Expression Purif., 41, 77-83 (2005)).

A final yield of the abundant culture by *P. methanolica* was 11.7 mg/l L-culture at maximum.

### 4-4. Evaluation of heat resistance:

Next, a recombinant BO by *P. methanolica* and a commercially available BO (manufactured by Amano Enzyme Inc.) were evaluated with respect to the heat resistance. The evaluation of the heat resistance was performed by the comparison in the residual activity after heating. For the measurement of the BO activity, ABTS was used as a substrate, a change in the absorbance at 730 nm with the progress of reaction (derived from an increase of the reaction product of ABTS) was followed. The measurement condition is shown in Table 12. During the activity measurement, the BO concentration was adjusted such that the change in the absorbance at 730 nm was from about 0.01 to 0.2 per minute. The reaction was started by adding an enzyme solution (5 to 20 µL) in an ABTS-containing phosphate buffer solution (2, 980 to 2,995 µL).

**Table 12**

| | |
|---|---|
| Buffer solution | 46.5 mM sodium phosphate aqueous solution (pH: 7.0) |
| ABTS concentration | 2 mM (final concentration) |
| O₂ concentration | Saturated with air (210·M, 25°C) |
| Reaction temperature | 25°C |

With respect to the 26 kinds in total of the heat-resistant BO mutant candidacies expressed by *P*. *methanolica* (Q49K, Q72E, V81L, Y121S, R147P, A185S, P210L, F225V, G258V, A264V, D322N, N335S, R356L, P359S, D370Y, V371A, P423L, M468V, L476P, V513L, A103P, Y270D, S299N, V381L, A418T and R437H) and a multiple mutant obtained by combining two, three or four of them, a heat resistance experiment was carried out. With respect to the heating of each enzyme solution, a method of rapidly moving 150 mL of an enzyme solution (100 mM potassium phosphate buffer (pH: 6.0)) as poured out into a 500-mL tube in an ice bath onto a heat block set up at 60°C, allowing it to stand for a fixed time and then rapidly again returning in an ice bath was employed. The results of this heat resistance verification experiment are summarized in Table 13.

### 4-5. Measurement of denaturation temperature:

The denaturation temperature Tₘ of the 55 kinds of heat-resistant BO mutants having been subjected to evaluation of heat resistance was measured by differential scanning calorimetry (hereinafter referred to as "DSC"). VP-DSC as manufactured by MicroCal, LLC was used for the DSC. An enzyme solution was used in an amount of from 2.0 to 2.5 mg/mL, and the temperature rise was carried out at a rate of 60°C per hour. The results are summarized along with the heat resistance verification experiment of the activity in Table 13.

**Table 13**

| Residual activity & denaturation temperature | Single mutant | Double mutant | Triple mutant or quartet mutant |
|---|---|---|---|
| 80 % or more & 77°C or higher | | Y121S/L476P, A264V/R356L, A264V/L476P, D322N/M468V | Q49K/V371AN513L, Y121S/D370Y/L476P, A185S/A264V/L476P, K225V/D322N/M468V, A264V/R356L/L476P, A264V/S299N/L476P, A264V/V381L/L476P, A264V/A418T/L476P, A264V/R437H/L476P, A103P/A264VN270D/L476P |
| 50 % or more & 75°C or higher | Q72E, V81 L, Y121 S, F225V, A264V, D322N, R356L, P359S, D370Y, P423L, M468V, L476P, A103P, S299N, V381L, A418T,R437H | | Q72E/P210L/A264V, V81 L/N335S/P423L, F225V/D370Y/L476P |
| 20 % or more & 72°C or higher | Q49K, R147P, A185S, P210L, G258V, N335S, V371A, V513T, V270D | Q49KN371A, Q72E/P210L, Q72E/A264V, V81 L/R147P, V81L/P423L, A185S/G258V, P210L/A264V, F225V/D322N, F225V/L476P. N335S/P423L, R356L/L476P, V371A/V513L | |
| Less than 20% & lower than 72°C | Wild type, commercial product | | |

The heat-resistant bilirubin oxidase mutant according to the embodiment of the present invention can be, for example, utilized as a catalyst for realizing an electrochemical four-electron reduction reaction of oxygen in a fuel cell using an electrode having an enzyme immobilized therein, especially on a cathode side of the enzyme cell.

It should be understood by those skilled in the art that various modifications, combinations, sub-combinations and alternations may occur depending on design requirements and other factors insofar as they are within the scope of the appended claims or the equivalents thereof.

## Claims

1. A heat-resistant bilirubin oxidase mutant obtained by deletion, replacement, addition or insertion of at least one amino acid residue of a wild type amino sequence of SEQ. ID. No. 1 of a bilirubin oxidase derived from an imperfect filamentous fungus, *Myrothecium verrucaria.*

2. The heat-resistant bilirubin oxidase mutant according to claim 1, wherein a denaturation temperature Tₘ value is 72°C or higher.

3. The heat-resistant bilirubin oxidase mutant according to claim 1, wherein a residual enzyme activity after heating at 60°C for one hour is 20 % or more.

4. The heat-resistant bilirubin oxidase mutant according to claim 1, wherein the imperfect filamentous fungus is a strain of *Myrothecium verrucaria* NBRC (IFO) 6113.

5. The heat-resistant bilirubin oxidase mutant according to claim 1, which is expressed by using a yeast, *Pichia methanolica* as a host.

6. The heat-resistant bilirubin oxidase mutant according to claim 1, which is represented by SEQ. ID. No. 2 wherein glutamine at the 49th position from the N-terminus of the wild type amino acid sequence is replaced with lysine (Q49K).

7. The heat-resistant bilirubin oxidase mutant according to claim 1, which is represented by SEQ. ID. No. 3 wherein glutamine at the 72nd position from the N-terminus of the wild type amino acid sequence is replaced with glutamic acid (Q72E).

8. The heat-resistant bilirubin oxidase mutant according to claim 1, which is represented by SEQ. ID. No. 4 wherein valine at the 81st position from the N-terminus of the wild type amino acid sequence is replaced with leucine (V81L) .

9. The heat-resistant bilirubin oxidase mutant according to claim 1, which is represented by SEQ. ID. No. 5 wherein tyrosine at the 121st position from the N-terminus of the wild type amino acid sequence is replaced with serine (Y121S).

10. The heat-resistant bilirubin oxidase mutant according to claim 1, which is represented by SEQ. ID. No. 6 wherein arginine at the 147th position from the N-terminus of the wild type amino acid sequence is replaced with proline (R147P) .

11. The heat-resistant bilirubin oxidase mutant according to claim 1, which is represented by SEQ. ID. No. 7 wherein alanine at the 185th position from the N-terminus of the wild type amino acid sequence is replaced with serine (A185S) .

12. The heat-resistant bilirubin oxidase mutant according to claim 1, which is represented by SEQ. ID. No. 8 wherein proline at the 210th position from the N-terminus of the wild type amino acid sequence is replaced with leucine (P210L).

13. The heat-resistant bilirubin oxidase mutant according to claim 1, which is represented by SEQ. ID. No. 9 wherein phenylalanine at the 225th position from the N-terminus of the wild type amino acid sequence is replaced with valine (F225V).

14. The heat-resistant bilirubin oxidase mutant according to claim 1, which is represented by SEQ. ID. No. 10 wherein glycine at the 258th position from the N-terminus of the wild type amino acid sequence is replaced with valine (G258V).

15. The heat-resistant bilirubin oxidase mutant according to claim 1, which is represented by SEQ. ID. No. 11 wherein alanine at the 264th position from the N-terminus of the wild type amino acid sequence is replaced with valine (A264V).

16. The heat-resistant bilirubin oxidase mutant according to claim 1, which is represented by SEQ. ID. No. 12 wherein aspartic acid at the 322nd position from the N-terminus of the wild type amino acid sequence is replaced with asparagine (D322N).

17. The heat-resistant bilirubin oxidase mutant according to claim 1, which is represented by SEQ. ID. No. 13 wherein asparagine at the 335th position from the N-terminus of the wild type amino acid sequence is replaced with serine (N335S).

18. The heat-resistant bilirubin oxidase mutant according to claim 1, which is represented by SEQ. ID. No. 14 wherein arginine at the 356th position from the N-terminus of the wild type amino acid sequence is replaced with leucine (R356L).

19. The heat-resistant bilirubin oxidase mutant according to claim 1, which is represented by SEQ. ID. No. 15 wherein proline at the 359th position from the N-terminus of the wild type amino acid sequence is replaced with serine (P359S).

20. The heat-resistant bilirubin oxidase mutant according to claim 1, which is represented by SEQ. ID. No. 16 wherein aspartic acid at the 370th position from the N-terminus of the wild type amino acid sequence is replaced with tyrosine (D370Y).

21. The heat-resistant bilirubin oxidase mutant according to claim 1, which is represented by SEQ. ID. No. 17 wherein valine at the 371st position from the N-terminus of the wild type amino acid sequence is replaced with alanine (V371A).

22. The heat-resistant bilirubin oxidase mutant according to claim 1, which is represented by SEQ. ID. No. 18 wherein proline at the 423rd position from the N-terminus of the wild type amino acid sequence is replaced with leucine (P423L).

23. The heat-resistant bilirubin oxidase mutant according to claim 1, which is represented by SEQ. ID. No. 19 wherein methionine at the 468th position from the N-terminus of the wild type amino acid sequence is replaced with valine (M468V).

24. The heat-resistant bilirubin oxidase mutant according to claim 1, which is represented by SEQ. ID. No. 20 wherein leucine at the 476th position from the N-terminus of the wild type amino acid sequence is replaced with proline (L476P) .

25. The heat-resistant bilirubin oxidase mutant according to claim 1, which is represented by SEQ. ID. No. 21 wherein valine at the 513rd position from the N-terminus of the wild type amino acid sequence is replaced with leucine (V513L) .

26. The heat-resistant bilirubin oxidase mutant according to claim 1, which is represented by SEQ. ID. No. 57 wherein alanine at the 103rd position from the N-terminus of the wild type amino acid sequence is replaced with proline (A103P).

27. The heat-resistant bilirubin oxidase mutant according to claim 1, which is represented by SEQ. ID. No. 58 wherein tyrosine at the 270th position from the N-terminus of the wild type amino acid sequence is replaced with aspartic acid (Y270D).

28. The heat-resistant bilirubin oxidase mutant according to claim 1, which is represented by SEQ. ID. No. 59 wherein serine at the 299th position from the N-terminus of the wild type amino acid sequence is replaced with asparagine (S299N) .

29. The heat-resistant bilirubin oxidase mutant according to claim 1, which is represented by SEQ. ID. No. 60 wherein valine at the 381st position from the N-terminus of the wild type amino acid sequence is replaced with leucine (V381L).

30. The heat-resistant bilirubin oxidase mutant according to claim 1, which is represented by SEQ. ID. No. 61 wherein alanine at the 418th position from the N-terminus of the wild type amino acid sequence is replaced with threonine (A418T).

31. The heat-resistant bilirubin oxidase mutant according to claim 1, which is represented by SEQ. ID. 62 wherein arginine at the 437th position from the N-terminus of the wild type amino acid sequence is replaced with histidine (R437H) .

32. A bilirubin oxidase multiple mutant comprising a combination of two or three or four amino acid residue replacements selected among the replacements according to claims 6 to 31.

33. The heat-resistant bilirubin oxidase mutant according to claim 32, which is represented by SEQ. ID. No. 22 wherein glutamine at the 49th position from the N-terminus of the wild type amino acid sequence is replaced with lysine (Q49K), and valine at the 371st position is replaced with alanine (V371A).

34. The heat-resistant bilirubin oxidase mutant according to claim 32, which is represented by SEQ. ID. No. 23 wherein glutamine at the 72nd position from the N-terminus of the wild type amino acid sequence is replaced with glutamic acid (Q72E), and proline at the 210th position is replaced with leucine (P210L).

35. The heat-resistant bilirubin oxidase mutant according to claim 32, which is represented by SEQ. ID. No. 24 wherein glutamine at the 72nd position from the N-terminus of the wild type amino acid sequence is replaced with glutamic acid (Q72E), and alanine at the 264th position is replaced with valine (A264V).

36. The heat-resistant bilirubin oxidase mutant according to claim 32, which is represented by SEQ. ID. No. 25 wherein valine at the 81st position from the N-terminus of the wild type amino acid sequence is replaced with leucine (V81L), and arginine at the 147th position is replaced with proline (R147P).

37. The heat-resistant bilirubin oxidase mutant according to claim 32, which is represented by SEQ. ID. No. 26 wherein valine at the 81st position from the N-terminus of the wild type amino acid sequence is replaced with leucine (V81L), and proline at the 423rd position is replaced with leucine (P423L).

38. The heat-resistant bilirubin oxidase mutant according to claim 32, which is represented by SEQ. ID. No. 27 wherein tyrosine at the 121st position from the N-terminus of the wild type amino acid sequence is replaced with serine (Y121S), and leucine at the 476th position is replaced with proline (L476P).

39. The heat-resistant bilirubin oxidase mutant according to claim 32, which is represented by SEQ. ID. No. 28 wherein alanine at the 185th position from the N-terminus of the wild type amino acid sequence is replaced with serine (A185S), and glycine at the 258th position is replaced with valine (G258V).

40. The heat-resistant bilirubin oxidase mutant according to claim 32, which is represented by SEQ. ID. No. 29 wherein proline at the 210th position from the N-terminus of the wild type amino acid sequence is replaced with leucine (P210L), and alanine at the 264th position is replaced with valine (A264V).

41. The heat-resistant bilirubin oxidase mutant according to claim 32, which is represented by SEQ. ID. No. 30 wherein phenylalanine at the 225th position from the N-terminus of the wild type amino acid sequence is replaced with valine (F225V), and aspartic acid at the 322nd position is replaced with asparagine (D322N).

42. The heat-resistant bilirubin oxidase mutant according to claim 32, which is represented by SEQ. ID. No. 31 wherein phenylalanine at 225th position from the N-terminus of the wild type amino acid sequence is replaced by valine (F225V), and leucine at the 476th position is replaced with proline (L476P).

43. The heat-resistant bilirubin oxidase mutant according to claim 32, which is represented by SEQ. ID. No. 32 wherein alanine at the 264th position from the N-terminus of the wild type amino acid sequence is replaced with valine (A264V), and arginine at the 356th position is replaced with leucine (R356L).

44. The heat-resistant bilirubin oxidase mutant according to claim 32, which is represented by SEQ. ID. No. 33 wherein alanine at the 264th position from the N-terminus of the wild type amino acid sequence is replaced with valine (A264V), and leucine at the 476th position is replaced with proline (L476P).

45. The heat-resistant bilirubin oxidase mutant according to claim 32, which is represented by SEQ. ID. No. 34 wherein aspartic acid at the 322nd position from the N-terminus of the wild type amino acid sequence is replaced with asparagine (D322N), and methionine at the 468th position is replaced with valine (M468V).

46. The heat-resistant bilirubin oxidase mutant according to claim 32, which is represented by SEQ. ID. No. 35 wherein asparagine at the 335th position from the N-terminus of the wild type amino acid sequence is replaced with serine (N335S), and proline at the 423rd position is replaced with leucine (P423L).

47. The heat-resistant bilirubin oxidase mutant according to claim 32, which is represented by SEQ. ID. No. 36 wherein arginine at the 356th position from the N-terminus of the wild type amino acid sequence is replaced with leucine (R356L), and leucine at the 476th position is replaced with proline (L476P).

48. The heat-resistant bilirubin oxidase mutant according to claim 32, which is represented by SEQ. ID. No. 37 wherein valine at the 371st position from the N-terminus of the wild type amino acid sequence is replaced with alanine (V371A), and valine at the 513rd position is replaced with leucine (V513L).

49. The heat-resistant bilirubin oxidase mutant according to claim 32, which is represented by SEQ. ID. No. 38 wherein glutamine at the 49th position from the N-terminus of the wild type amino acid sequence is replaced with lysine (Q49K), valine at the 371st position is replaced with alanine (V371A), and valine at the 513rd position is replaced with leucine (V513L).

50. The heat-resistant bilirubin oxidase mutant according to claim 32, which is represented by SEQ. ID. No. 39 wherein glutamine at the 72nd position from the N-terminus of the wild type amino acid sequence is replaced with glutamic acid (Q72E), proline at the 210th position is replaced with leucine (P210L), and alanine at the 264th position is replaced with valine (A264V).

51. The heat-resistant bilirubin oxidase mutant according to claim 32, which is represented by SEQ. ID. No. 40 wherein valine at the 81st position from the N-terminus of the wild type amino acid sequence is replaced with leucine (V81L), asparagine at the 335th position is replaced with serine (N335S), and proline at the 423rd position is replaced with leucine (P423L).

52. The heat-resistant bilirubin oxidase mutant according to claim 32, which is represented by SEQ. ID. No. 41 wherein tyrosine at the 121st position from the N-terminus of the wild type amino acid sequence is replaced with serine (Y121S), aspartic acid at the 370th position is replaced with tyrosine (D370Y), and leucine at the 476th position is replaced with proline (L476P).

53. The heat-resistant bilirubin oxidase mutant according to claim 32, which is represented by SEQ. ID. No. 42 wherein alanine at the 185th position from the N-terminus of the wild type amino acid sequence is replaced with serine (A185S), alanine at the 264th position is replaced with valine (A264V), and leucine at the 476th position is replaced with proline (L476P).

54. The heat-resistant bilirubin oxidase mutant according to claim 32, which is represented by SEQ. ID. No. 43 wherein phenylalanine at the 225th position from the N-terminus of the wild type amino acid sequence is replaced with valine (F225V), aspartic acid at the 322nd position is replaced with asparagine (D322N), and methionine at the 468th position is replaced with valine (M468V).

55. The heat-resistant bilirubin oxidase mutant according to claim 32, which is represented by SEQ. ID. No. 44 wherein phenylalanine at the 225th position from the N-terminus of the wild type amino acid sequence is replaced with valine (F225V), aspartic acid at the 370th position is replaced with tyrosine (D370Y), and leucine at the 476th position is replaced with proline (L476P).

56. The heat-resistant bilirubin oxidase mutant according to claim 32, which is represented by SEQ. ID. No. 45 wherein alanine at the 264th position from the N-terminus of the wild type amino acid sequence is replaced with valine (A264V), arginine at the 356th position is replaced with leucine (R356L), and leucine at the 476th position is replaced with proline (L476P).

57. The heat-resistant bilirubin oxidase mutant according to claim 32, which is represented by SEQ. ID. No. 63 wherein alanine at the 264th position from the N-terminus of the wild type amino acid sequence is replaced with valine (A264V), serine at the 299th position is replaced with asparagine (S299N), and leucine at the 476th position is replaced with proline (L476P).

58. The heat-resistant bilirubin oxidase mutant according to claim 32, which is represented by SEQ. ID. No. 64 wherein alanine at the 264th position from the N-terminus of the wild type amino acid sequence is replaced with valine (A264V), valine at the 381st position is replaced with leucine (V381L), and leucine at the 476th position is replaced with proline (L476P).

59. The heat-resistant bilirubin oxidase mutant according to claim 32, which is represented by SEQ. ID. No. 65 wherein alanine at the 264th position from the N-terminus of the wild type amino acid sequence is replaced with valine (A264V), alanine at the 418th position is replaced with threonine (A418T), and leucine at the 476th position is replaced with proline (L476P).

60. The heat-resistant bilirubin oxidase mutant according to claim 32, which is represented by SEQ. ID. No. 66 wherein alanine at the 264th position from the N-terminus of the wild type amino acid sequence is replaced with valine (A264V), arginine at the 437th position is replaced with histidine (R437H), and leucine at the 476th position is replaced with proline (L476P).

61. The heat-resistant bilirubin oxidase mutant according to claim 32, which is represented by SEQ. ID. No. 67 wherein alanine at the 103rd position from the N-terminus of the wild type amino acid sequence is replaced with proline (A103P), alanine at the 264th position from the N-terminus is replaced with valine (A264V), tyrosine at the 270th position is replaced with aspartic acid (Y270D), and leucine at the 476th position is replaced with proline (L476P).
